# EUROPÄISCHE PATENTANMELDUNG

(11) **EP 4 640 258 A1**
(43) Veröffentlichungstag der Anmeldung: **29.10.2025**
(21) Anmeldenummer: 25171762.5
(22) Anmeldetag: 22.04.2025
(51) Int. Cl.: A61M 16/00, F16M 11/22, F16M 11/04

(54) **BEATMUNGSGERÄT MIT DISPLAY**

(30) Priorität: 25.04.2024 DE 102024111636
(71) Anmelder: Löwenstein Medical Technology S.A., 2557 Luxembourg (LU)
(72) Erfinder: Mehr, Fritz, 35799 Merenberg (DE)
(74) Vertreter: Löwenstein Medical IP

(57) **Zusammenfassung**

Ein Beatmungsgerät umfasst: ein Hauptgehäuse; eine Displayeinheit mit einem Display und einer Displayrückwand, die eine an einer Rückseite des Displays befestigte Grundplatte und mehrere von der Grundplatte abstehende erste Verschraubungsabschnitte (19) umfasst; ein Displaygehäuse (9), das schalenartig mit einer Schalenöffnung zum Einführen der Displayeinheit ausgebildet ist, wobei die Displayeinheit so vom Displaygehäuse aufgenommen ist, dass das Display die Schalenöffnung mit seiner der Rückseite gegenüberliegenden Vorderseite zumindest teilweise abdeckt; einen Displayhalter, der das Displaygehäuse mechanisch mit dem Hauptgehäuse koppelt; wobei das Displaygehäuse in seinem Inneren eine mit einer Innenwand des Displaygehäuses verbundene Versteifungsstruktur aufweist, wobei die Versteifungsstruktur mehrere zweite Verschraubungsabschnitte (25) umfasst, die so angeordnet sind, dass sie jeweils mindestens einem der ersten Verschraubungsabschnitte in einem Überlappungsbereich gegenüberliegen, wenn die Displayeinheit vollständig ins Displaygehäuse eingeführt ist, wobei die ersten und die zweiten Verschraubungsabschnitte in jedem der Überlappungsbereiche mittels mindestens eines Schraubelements (29) miteinander verschraubt sind, sodass die Displayeinheit am Displaygehäuse fixiert ist, wobei jedes der Schraubelemente vollständig im Inneren des Displaygehäuses angeordnet ist, wobei in einer Außenwand des Displaygehäuses mehrere Zugangsöffnungen (31) so ausgebildet sind, dass jedes der Schraubelemente über eine der Zugangsöffnungen von außerhalb des Displaygehäuses für ein Schraubwerkzeug zugänglich ist.

## Beschreibung

### Stand der Technik

Ein Beatmungsgerät kann ein Hauptgehäuse zum Aufnehmen von für einen Beatmungsbetrieb erforderlichen Komponenten und ein am Hauptgehäuse befestigtes Display zum Anzeigen beatmungsrelevanter Informationen umfassen. Da das Display im Betrieb oft von verschiedenen Personen angefasst wird, ist aus hygienischen Gründen eine regelmäßige Reinigung des Displays erforderlich. Das Display sollte daher eine möglichst glatte, abgerundete Außenfläche haben, damit sich beim Abwischen der Außenfläche kein Schmutz und/oder keine Flüssigkeit an Unebenheiten wie Stufen, Kanten, Ritzen oder Löchern sammeln kann.

### Offenbarung der Erfindung

Eine Aufgabe der Erfindung kann darin gesehen werden, ein Beatmungsgerät mit einem Display bereitzustellen, dessen Außenfläche in effizienter Weise gereinigt werden kann.

Diese Aufgabe wird durch den Gegenstand des unabhängigen Anspruchs gelöst. Vorteilhafte Ausführungsformen der Erfindung sind in den abhängigen Ansprüchen, der nachfolgenden Beschreibung und den beigefügten Figuren dargelegt.

Die Erfindung betrifft ein Beatmungsgerät. Das Beatmungsgerät umfasst: ein Hauptgehäuse zum Aufnehmen von Komponenten, die für einen Beatmungsbetrieb des Beatmungsgeräts erforderlich sind, wobei das Hauptgehäuse einen Atemgasanschluss zum Anschließen einer Atemgasquelle und/oder eines zu einem Patienten führenden Schlauchsystems umfasst; eine Displayeinheit mit einem Display zum Anzeigen beatmungsrelevanter Informationen und einer Displayrückwand, die eine an einer Rückseite des Displays befestigte Grundplatte und mehrere von der Grundplatte abstehende erste Verschraubungsabschnitte umfasst; ein Displaygehäuse, das schalenartig mit einer Schalenöffnung zum Einführen der Displayeinheit ausgebildet ist, wobei die Displayeinheit so vom Displaygehäuse aufgenommen ist, dass das Display die Schalenöffnung mit seiner der Rückseite gegenüberliegenden Vorderseite zumindest teilweise abdeckt; einen Displayhalter, der das Displaygehäuse mechanisch mit dem Hauptgehäuse koppelt. Das Displaygehäuse weist in seinem Inneren eine mit einer Innenwand des Displaygehäuses verbundene Versteifungsstruktur zum Versteifen des Displaygehäuses auf. Die Versteifungsstruktur umfasst mehrere zweite Verschraubungsabschnitte, die so angeordnet sind, dass sie jeweils mindestens einem der ersten Verschraubungsabschnitte in einem Überlappungsbereich gegenüberliegen, wenn die Displayeinheit vollständig ins Displaygehäuse eingeführt ist. Die ersten und die zweiten Verschraubungsabschnitte sind in jedem der Überlappungsbereiche mittels mindestens eines Schraubelements miteinander verschraubt, sodass die Displayeinheit am Displaygehäuse fixiert ist. Dabei ist jedes der Schraubelemente vollständig im Inneren des Displaygehäuses angeordnet. In einer Außenwand des Displaygehäuses sind mehrere Zugangsöffnungen so ausgebildet, dass jedes der Schraubelemente über eine der Zugangsöffnungen von außerhalb des Displaygehäuses für ein Schraubwerkzeug zugänglich ist.

Aufgrund der schalenartigen Form der Displayeinheit und der innenliegenden Verschraubungsstellen kann verhindert werden, dass sich beim Abwischen der Displayeinheit an bestimmten Stellen übermäßig Schmutz oder Flüssigkeit sammelt. Vorteilhafterweise kann dies mithilfe eines besonders einfachen und dennoch robusten Aufbaus des Beatmungsgeräts, insbesondere des Displaygehäuses und/oder der Displayeinheit, erreicht werden.

Das Beatmungsgerät kann zur invasiven und/oder nicht invasiven Beatmung eines Patienten ausgebildet sein.

Der Atemgasanschluss kann mehrere separate Ein- und/oder Ausgänge, beispielsweise einen Atemgaseingang und einen Atemgasausgang, umfassen. Besonders günstig ist es, wenn der Atemgasanschluss teilweise oder vollständig in eine Gehäusefront des Hauptgehäuses eingebaut ist.

Unter "Atemgasquelle" kann beispielsweise eine Gasflasche, ein Atemgasmischer, ein Atemgasleitungssystem (z. B. in einem Klinikgebäude) oder eine Kombination aus mindestens zwei dieser Beispiele verstanden werden.

Unter "Schlauchsystem" kann eine Anordnung aus einem oder mehreren Schläuchen zum fluidischen Verbinden des Beatmungsgeräts mit einer Patientenschnittstelle, beispielsweise einer Beatmungsmaske, einem Tubus oder einer Nasenbrille, verstanden werden.

Unter "Display" kann beispielsweise ein LC- oder ein OLED-Display verstanden werden.

Das Display kann mit seiner Vorderseite die Schalenöffnung auch vollständig abdecken. Dabei kann eine gesamte (einem Betrachter des Displays zugewandte) Vorderseite der ins Displaygehäuse eingeführten Displayeinheit durch die Vorderseite des Displays, insbesondere durch eine Vorderseite einer Displayscheibe aus Glas und/oder Kunststoff, gebildet sein.

Unter "Grundplatte" kann allgemein eine platten- oder gitterartige Stützstruktur zum Stützen des Displays verstanden werden. Beispielsweise kann die Grundplatte aus Metall und/oder Kunststoff gefertigt sein. Die Grundplatte kann insbesondere als ein Blech- und/oder Schweißteil ausgeführt sein.

Die ersten Verschraubungsabschnitte können jeweils schräg oder senkrecht von der Grundplatte abstehen. Beispielsweise können die ersten Verschraubungsabschnitte zumindest teilweise durch entsprechendes Umbiegen von Abschnitten der Grundplatte gefertigt sein. Zusätzlich oder alternativ kann es sich bei den ersten Verschraubungsabschnitten zumindest teilweise um mit der Grundplatte stoffschlüssig, insbesondere durch Schweißen und/oder Löten, verbundene plattenförmige Abschnitte, beispielsweise Blechabschnitte, handeln.

Bei dem schalenartigen Displaygehäuse kann es sich beispielsweise um ein Gehäuse oder ein Gehäuseteil mit einer verrundeten Außenfläche handeln. Anders ausgedrückt kann das Displaygehäuse so geformt sein, dass seine Außenfläche keine übermäßig scharfen Kanten und/oder keine übermäßig spitzen Ecken aufweist. Auf diese Weise können unerwünschte stufenartige Übergänge an der Außenfläche vermieden werden. Das Displaygehäuse kann aus Kunststoff und/oder Metall gefertigt sein. Beispielsweise kann das Displaygehäuse zusammen mit der Versteifungsstruktur als ein einziges Teil (z. B. als ein einziges Spritzgießteil) und/oder aus demselben Material (z. B. aus demselben Kunststoffmaterial) gefertigt sein.

Der Displayhalter kann beispielsweise ausgebildet sein, um das Displaygehäuse beweglich mit dem Hauptgehäuse zu koppeln, sodass das Displaygehäuse in seiner Position und/oder Orientierung relativ zum Hauptgehäuse angepasst und in der angepassten Position und/oder Orientierung fixiert werden kann. Beispielsweise kann der Displayhalter einen speziellen Friktionsmechanismus zum (beispielsweise stufenlosen) Einstellen einer Reibung zwischen dem Displaygehäuse und dem Hauptgehäuse umfassen. Vorzugsweise kann die Reibung mittels des Friktionsmechanismus so einstellbar sein, dass das Displaygehäuse mit einem gewissen Kraftaufwand manuell in eine gewünschte Position und/oder Orientierung bewegt werden kann und beim Loslassen allein aufgrund der vom Friktionsmechanismus erzeugten Reibung in der gewünschten Position und/oder Orientierung verbleibt.

Zweckmäßigerweise kann der Displayhalter das Displaygehäuse im betriebsfähigen Zustand des Beatmungsgeräts gegenüber einer Oberseite des Hauptgehäuses halten. Möglich ist aber auch eine seitliche Anordnung des Displaygehäuses.

Die Versteifungsstruktur kann beispielsweise eine Mehrzahl von mit der Innenwand des Displaygehäuses verbundenen Versteifungsrippen umfassen. Die Versteifungsrippen können parallel und/oder orthogonal zueinander ausgerichtet sein. Anders ausgedrückt können die Versteifungsrippen Längs- und/oder Querrippen umfassen. Zusätzlich oder alternativ ist eine schräge Ausrichtung der Versteifungsrippen zueinander möglich. Die Versteifungsrippen können zumindest teilweise in ihrer jeweiligen Längsrichtung betrachtet gekrümmt und/oder gerade verlaufen. Beispielsweise können die Versteifungsrippen zumindest teilweise kreis-, kreissegment- oder ringförmig gebogen sein.

Beispielsweise können sich die ersten und die zweiten Verschraubungsabschnitte jeweils paarweise in einem Überlappungsbereich gegenüberliegen. Anders ausgedrückt kann jeder der Überlappungsbereiche jeweils genau einen der ersten Verschraubungsabschnitte und genau einen der zweiten Verschraubungsabschnitte umfassen. Möglich sind aber auch Überlappungsbereiche mit mehr als zwei Verschraubungsabschnitten, beispielsweise mit mindestens einem ersten Verschraubungsabschnitt und mindestens zwei zweiten Verschraubungsabschnitten oder mit mindestens zwei ersten Verschraubungsabschnitten und mindestens einem zweiten Verschraubungsabschnitt. Beispielsweise kann im ersten Fall der erste Verschraubungsabschnitt zwischen den zweiten Verschraubungsabschnitten und/oder im zweiten Fall der zweite Verschraubungsabschnitt zwischen den ersten Verschraubungsabschnitten angeordnet sein.

Unter "Schraubelement" kann beispielsweise eine Schraube, ein Gewindebolzen, eine Schraubenmutter oder eine Kombination aus mindestens zwei dieser Beispiele verstanden werden.

Es ist möglich, dass jede der Zugangsöffnungen zu einem der Zugangsöffnung zugewandten Ende des jeweiligen Schraubelements in einem definierten Abstand angeordnet ist. Dies ermöglicht es, die Schraubelemente besonders tief im Inneren des Displaygehäuses anzuordnen, sodass die Schraubelemente von außen nicht oder nur bei genauerem Hinsehen zu erkennen sind.

Um eine besonders stabile Fixierung der Displayeinheit zu ermöglichen, kann das Beatmungsgerät mindestens vier verschiedene Überlappungsbereiche umfassen.

Im Folgenden werden verschiedene Ausführungsformen der Erfindung beschrieben. Diese Ausführungsformen sind nicht als Beschränkung des Umfangs der Erfindung zu verstehen.

Gemäß einer Ausführungsform kann ein Abstand einer jeden der Zugangsöffnungen zu einem der Zugangsöffnung zugewandten Ende des jeweiligen Schraubelements mindestens 1 cm, mindestens 2 cm, mindestens 5 cm oder mindestens 10 cm betragen.

Gemäß einer Ausführungsform können die Schraubelemente mehrere Schrauben umfassen, wobei jede der Schrauben durch ein Loch in einem der ersten Verschraubungsabschnitte und/oder durch ein Loch in einem der zweiten Verschraubungsabschnitte geführt ist und/oder mit einer Schraubenmutter, die an einem der ersten Verschraubungsabschnitte und/oder an einem der zweiten Verschraubungsabschnitte angeordnet ist, verschraubt ist. Beispielsweise kann es sich bei dem der Zugangsöffnung zugewandten Ende des Schraubelements um einen Schraubenkopf der jeweiligen Schraube handeln.

Gemäß einer Ausführungsform kann das Displaygehäuse einen Schalenboden und eine mit dem Schalenboden verbundene Schalenseitenwand umfassen. Die Schalenöffnung kann durch einen (vom Schalenboden abgewandten) umlaufenden Rand der Schalenseitenwand begrenzt sein. Ferner kann das Displaygehäuse in Richtung seiner Höhe (im betriebsfähigen Zustand des Beatmungsgeräts) einerseits durch einen oberen Abschnitt der Schalenseitenwand und andererseits durch einen unteren Abschnitt der Schalenseitenwand begrenzt sein. Zusätzlich oder alternativ kann das Displaygehäuse in Richtung seiner Breite, d. h. in einer Richtung quer zu seiner Höhe (im betriebsfähigen Zustand des Beatmungsgeräts), einerseits durch einen linken Abschnitt der Schalenseitenwand und andererseits durch einen rechten Abschnitt der Schalenseitenwand begrenzt sein. Dabei können die Zugangsöffnungen in mindestens einem der Abschnitte der Schalenseitenwand ausgebildet sein. Alternativ ist es möglich, dass mindestens eine der Zugangsöffnungen im Schalenboden ausgebildet ist.

Zusätzlich oder alternativ kann das Displaygehäuse in Richtung seiner Tiefe, d. h. in einer Richtung quer zu seiner Höhe und Breite (im betriebsfähigen Zustand des Beatmungsgeräts), einerseits durch den Schalenboden und andererseits durch das Display, insbesondere durch die Displayrückwand, begrenzt sein.

Gemäß einer Ausführungsform können die Zugangsöffnungen außerhalb des oberen Abschnitts der Schalenseitenwand ausgebildet sein. Dadurch kann verhindert werden, dass Schmutz oder Flüssigkeit beim Reinigen des Displaygehäuses aufgrund der Schwerkraft durch die Zugangsöffnungen ins Innere des Displaygehäuses gelangt.

Gemäß einer Ausführungsform können die Zugangsöffnungen nur in einem der Abschnitte der Schalenseitenwand, insbesondere nur in deren unterem Abschnitt, ausgebildet sein. Somit brauchen die Schraubelemente nur an einer Seite des Displaygehäuses eingeschraubt bzw. gelöst zu werden, um die Displayeinheit zu montieren bzw. zu demontieren. Dies vereinfacht die Wartung, Instandsetzung und Reparatur der Displayeinheit im Vergleich zu einer Ausführungsform, bei der die Schraubelemente an verschiedenen Seiten der Schalenseitenwand angeordnet sind. Sind die Zugangsöffnungen nur im unteren Abschnitt der Schalenseitenwand ausgebildet, so hat dies den Vorteil, dass die Zugangsöffnungen im betriebsfähigen Zustand des Beatmungsgeräts nicht oder allenfalls bei sehr genauem Hinsehen von außen erkennbar sind, was zu einem ästhetischeren Erscheinungsbild des Beatmungsgeräts beiträgt. Zudem wird auf diese Weise wirksam verhindert, dass Schmutz oder Flüssigkeit beim Reinigen des Displaygehäuses durch die Zugangsöffnungen ins Innere des Displaygehäuses gelangt.

Gemäß einer Ausführungsform kann die Versteifungsstruktur eine Mehrzahl länglicher Versteifungsrippen umfassen. In diesem Fall können die Versteifungsrippen jeweils einerseits mit der Schalenseitenwand und andererseits mit dem Schalenboden verbunden sein. Anders ausgedrückt können die Versteifungsrippen ausbildet sein, um die Schalenseitenwand am Schalenboden abzustützen (und umgekehrt).

Gemäß einer Ausführungsform können die Versteifungsrippen eine Mehrzahl parallel zueinander angeordneter erster Versteifungsrippen und eine Mehrzahl parallel zueinander angeordneter zweiter Versteifungsrippen umfassen. In diesem Fall können die ersten Versteifungsrippen in ihrer Längsrichtung betrachtet jeweils orthogonal und/oder schräg zu den zweiten Versteifungsrippen ausgerichtet sein. Anders ausgedrückt kann die Längsrichtung einer jeden der ersten Versteifungsrippen orthogonal oder schräg zur Längsrichtung einer jeden der zweiten Versteifungsrippen verlaufen. Die Versteifungsrippen können darüber hinaus eine Mehrzahl parallel zueinander angeordneter weiterer Versteifungsrippen umfassen, die in ihrer Längsrichtung betrachtet jeweils orthogonal und/oder schräg zu den ersten Versteifungsrippen und/oder den zweiten Versteifungsrippen ausgerichtet sein können. Wie weiter oben erwähnt, können die Versteifungsrippen zumindest teilweise in ihrer jeweiligen Längsrichtung betrachtet gekrümmt und/oder gerade verlaufen. Beispielsweise können die Versteifungsrippen zumindest teilweise kreis-, kreissegment- oder ringförmig gebogen sein.

Gemäß einer Ausführungsform können die ersten Versteifungsrippen jeweils einerseits mit dem oberen Abschnitt und/oder dem unteren Abschnitt der Schalenseitenwand und andererseits mit dem Schalenboden verbunden sein. Dabei können sich die ersten Versteifungsrippen in ihrer Längsrichtung betrachtet jeweils über einen Teil der Höhe des Displaygehäuses oder über dessen gesamte Höhe erstrecken.

Gemäß einer Ausführungsform können die zweiten Versteifungsrippen jeweils einerseits mit dem linken Abschnitt und/oder dem rechten Abschnitt der Schalenseitenwand und andererseits mit dem Schalenboden verbunden sein. Dabei können sich die zweiten Versteifungsrippen in ihrer Längsrichtung betrachtet jeweils über einen Teil der Breite des Displaygehäuses oder über dessen gesamte Breite erstrecken.

Gemäß einer Ausführungsform kann mindestens eine der ersten Versteifungsrippen zusätzlich mit mindestens einer der zweiten Versteifungsrippen verbunden sein. Dies kann die Stabilität des Displaygehäuses weiter erhöhen.

Beispielsweise kann mindestens eine der ersten Versteifungsrippen nur mit dem oberen Abschnitt der Schalenseitenwand und dem Schalenboden (und zusätzlich mit mindestens einer der zweiten Versteifungsrippen) und mindestens eine weitere der ersten Versteifungsrippen nur mit dem unteren Abschnitt der Schalenseitenwand und dem Schalenboden (und zusätzlich mit mindestens einer der zweiten Versteifungsrippen) verbunden sein.

Zusätzlich oder alternativ kann mindestens eine der zweiten Versteifungsrippen nur mit dem linken Abschnitt der Schalenseitenwand und dem Schalenboden (und zusätzlich mit mindestens einer der ersten Versteifungsrippen) verbunden sein und mindestens eine weitere der zweiten Versteifungsrippen nur mit dem rechten Abschnitt der Schalenseitenwand und dem Schalenboden (und zusätzlich mit mindestens einer der ersten Versteifungsrippen) verbunden sein.

Gemäß einer Ausführungsform kann eine jeweilige Länge der Versteifungsrippen höchstens 30 %, höchstens 50 % oder höchstens 70 % der Höhe und/oder Breite des Displaygehäuses betragen.

Gemäß einer Ausführungsform können die Versteifungsrippen zumindest teilweise unterschiedlich lang und/oder zumindest teilweise gleich lang sein. Beispielsweise können sich die ersten Versteifungsrippen in ihrer Länge zumindest teilweise voneinander und/oder zumindest teilweise von den zweiten Versteifungsrippen unterscheiden. Zusätzlich oder alternativ können sich die zweiten Versteifungsrippen in ihrer Länge zumindest teilweise voneinander unterscheiden.

Gemäß einer Ausführungsform kann das Displaygehäuse in seinem Inneren einen von den Versteifungsrippen freien Displayaufnahmebereich zum Aufnehmen zumindest eines Abschnitts der Displayeinheit aufweisen. Dies ermöglicht eine besonders flache und dennoch stabile Bauform des Displaygehäuses. Eine Grundfläche des Displayaufnahmebereichs kann beispielsweise zwischen 20 % und 70 %, insbesondere zwischen 20 % und 50 %, einer Grundfläche des Schalenbodens betragen.

Gemäß einer Ausführungsform kann mindestens einer oder jeder der zweiten Verschraubungsabschnitte ein (beispielsweise platten- oder blechartiger) Abschnitt von einer der Versteifungsrippen sein.

Gemäß einer Ausführungsform können im Inneren des Displaygehäuses mehrere Schächte zum Führen des Schraubwerkzeugs ausgebildet sein. Jeder der Schächte kann sich ausgehend von einer der Zugangsöffnungen hin zu dem der Zugangsöffnung zugewandten Ende des jeweiligen Schraubelements und/oder hin zu einem der Überlappungsbereiche erstrecken. In diesem Fall kann eine Schachtwand eines jeden der Schächte so ausgebildet sein, dass ein im Schacht befindliches Schraubelement nicht aus dem Schacht heraus ins Innere des Displaygehäuses fallen kann, etwa bei der Montage oder Demontage der Displayeinheit. Beispielsweise kann die Schachtwand zu diesem Zweck geschlossen sein.

Möglich ist auch eine nicht geschlossene Schachtwand mit entsprechend kleinen Öffnungen, beispielsweise eine zumindest teilweise gitter- oder netzartig ausgebildete Schachtwand. Unter "Schacht" kann beispielsweise ein rohr- oder rinnenförmiges Element verstanden werden. Es ist zweckmäßig, wenn der Schacht in seiner Längsrichtung betrachtet geradlinig oder nur geringfügig gekrümmt verläuft, um das Einführen eines länglichen Schraubwerkzeugs, beispielsweise einer Klinge eines Schraubenziehers, zu ermöglichen. Beispielsweise kann die Schachtwand mindestens einen der folgenden Abschnitte umfassen: einen Abschnitt des Schalenbodens, einen Abschnitt der Schalenseitenwand, einen Abschnitt der Versteifungsstruktur (insbesondere einer Versteifungsrippe), einen Abschnitt der Displayrückwand (insbesondere der Grundplatte). Diese Ausführungsform ermöglicht es, das Schraubwerkzeug blind über die jeweilige Zugangsöffnung zu einem der Schraubelemente zu führen. Dies kann die Montage bzw. Demontage deutlich vereinfachen.

Beispielsweise kann die Schachtwand Abschnitte von mindestens zwei parallel zueinander angeordneten ersten oder zweiten Versteifungsrippen umfassen.

Gemäß einer Ausführungsform kann das Display an seiner Vorderseite eine Displayscheibe aus Glas und/oder Kunststoff aufweisen. In diesem Fall kann die Displayscheibe die Schalenöffnung größtenteils oder vollständig abdecken. Beispielsweise kann die Displayscheibe in Richtung der Höhe und/oder Breite des Displaygehäuses betrachtet bündig mit einem umlaufenden Rand der Schalenöffnung abschließen. Die Displayeinheit kann ferner ein Bedienelement umfassen, das an und/oder in einem Scheibenloch in der Displayscheibe relativ zur Displayscheibe dreh- und/oder verschiebbar gelagert ist. Unter "Scheibenloch" kann eine von einem umlaufenden Rand begrenzte Durchgangsöffnung im Material der Displayscheibe verstanden werden. Anders ausgedrückt kann das Scheibenloch eine Vorderseite der Displayscheibe mit deren Rückseite verbinden. Das Scheibenloch kann beispielsweise kreisrund, oval oder länglich (d. h. ein Langloch) sein.

Gemäß einer Ausführungsform kann das Bedienelement mittels eines zusätzlichen Schraubelements an einem ersten Ende eines länglichen Koppelelements fixiert sein, wobei ein zweites Ende des Koppelelements dreh- und/oder verschiebbar am und/oder im Displaygehäuse gelagert sein kann. Das zusätzliche Schraubelement kann vollständig im Inneren des Displaygehäuses angeordnet sein und in der Außenwand des Displaygehäuses kann eine zusätzliche Zugangsöffnung so ausgebildet sein, dass das zusätzliche Schraubelement in einer bestimmten Position und/oder Orientierung des Bedienelements relativ zur Displayscheibe über die zusätzliche Zugangsöffnung von außerhalb des Displaygehäuses für ein Schraubwerkzeug zugänglich ist. Dabei kann ein Abstand der zusätzlichen Zugangsöffnung zu einem der zusätzlichen Zugangsöffnung zugewandten Ende des zusätzlichen Schraubelements mindestens 1 cm, mindestens 2 cm, mindestens 5 cm oder mindestens 10 cm betragen. Unter "Koppelelement" kann ein stabförmiges Element wie beispielsweise ein Bolzen, ein Stift oder ein Rohr verstanden werden. Es ist möglich, dass das Bedienelement durch das Scheibenloch ragt und/oder das Koppelelement durch das Scheibenloch ragt. Die zusätzliche Zugangsöffnung kann beispielsweise in einem der Abschnitte der Schalenseitenwand, insbesondere in deren unterem Abschnitt, ausgebildet sein. Das Bedienelement kann auch mittels mehrerer zusätzlicher Schraubelemente am ersten Ende des Koppelelements fixiert sein. In der Außenwand des Displaygehäuses können auch mehrere zusätzliche Zugangsöffnungen ausgebildet sein.

Gemäß einer Ausführungsform kann das erste Ende des Koppelelements in eine Ausnehmung des Bedienelements eingeführt und mittels des zusätzlichen Schraubelements in der Ausnehmung fixiert sein. Unter "Ausnehmung" kann beispielsweise ein becher- oder buchsenförmiger Abschnitt des Bedienelements, insbesondere eines zylinderförmigen Grundkörpers des Bedienelements, wie er nachstehend beschrieben wird, verstanden werden. Das erste Ende des Koppelelements kann beispielsweise derart formschlüssig in die Ausnehmung eingreifen, dass ein Verrutschen des in die Ausnehmung eingeführten Koppelelements relativ zur Ausnehmung um und/oder entlang deren Mittelachse mit normalem Kraftaufwand nicht möglich ist. Mittels des zusätzlichen Schraubelements kann in diesem Fall eine zusätzliche Haltekraft auf das Koppelelement ausgeübt werden.

Gemäß einer Ausführungsform kann das Bedienelement einen zylinderförmigen Grundkörper und einen von einer äußeren Mantelfläche des Grundkörpers abstehenden Kragen mit einer an eine (beispielsweise ringförmige) Innenkontur des Scheibenlochs angepassten (beispielsweise ringförmigen) Außenkontur umfassen. In diesem Fall kann der Grundkörper durch das Scheibenloch geführt sein, sodass er beidseitig von der Displayscheibe absteht. Zudem kann der Kragen ins Scheibenloch eingeführt sein, sodass die Außenkontur zumindest teilweise von der Innenkontur umgeben ist. Der Kragen kann zusätzlich als ein Anschlag zum Festlegen des Grundkörpers (oder des Bedienelements) in mindestens einer Richtung parallel zu einer Mittelachse des Scheibenlochs ausgebildet sein. Der Kragen kann die äußere Mantelfläche des Grundkörpers in deren Umfangsrichtung teilweise oder vollständig umlaufen.

Gemäß einer Ausführungsform kann der Kragen bündig mit der Vorderseite des Displays abschließen. Die Vorderseite des Displays kann insbesondere durch eine (ebene) Außenfläche der Displayscheibe gebildet sein. In diesem Fall kann der Kragen bündig mit der ans Scheibenloch grenzenden Außenfläche der Displayscheibe abschließen, sodass ein Übergang zwischen dem Kragen und der Außenfläche der Displayscheibe im Wesentlichen stufenlos ist. Dadurch kann verhindert werden, dass sich beim Reinigen der Displayscheibe Schmutz oder Flüssigkeit am Übergang sammelt.

Gemäß einer Ausführungsform kann ein (beispielsweise ringförmiger) Spalt zwischen dem Kragen und der Displayscheibe, insbesondere zwischen der Außenkontur des Kragens und der Innenkontur des Scheibenlochs, fluiddicht verschlossen sein. Dazu kann der Spalt entsprechend klein dimensioniert und/oder mit einem geeigneten Dichtmaterial ausgefüllt sein. Dies verhindert das Eindringen von Schmutz oder Flüssigkeit ins Innere der Displayeinheit und/oder des Displaygehäuses.

Gemäß einer Ausführungsform kann der Kragen die Displayscheibe, insbesondere einen das Scheibenloch begrenzenden Randabschnitt der Displayscheibe, an einer ringförmigen Berührungsfläche berühren.

Gemäß einer Ausführungsform können sich die Innenkontur des Scheibenlochs und die Außenkontur des Kragens an einer ringförmigen Berührungsfläche berühren.

Dies verhindert das Eindringen von Schmutz oder Flüssigkeit ins Innere der Displayeinheit und/oder des Displaygehäuses, ohne dass zusätzlich Dichtmaterial in einen etwaigen Spalt zwischen dem Kragen und der Displayscheibe bzw. zwischen der Innenkontur und der Außenkontur eingebracht werden muss.

Gemäß einer Ausführungsform kann das zusätzliche Schraubelement in eine Durchgangsöffnung, die die äußere Mantelfläche des Grundkörpers mit einer inneren Mantelfläche des Grundkörpers und/oder mit einer inneren Mantelfläche der Ausnehmung verbindet, eingeführt (beispielsweise geschraubt) sein, insbesondere so weit, dass das zusätzliche Schraubelement über die innere Mantelfläche des Grundkörpers hinaus ins Innere des Grundkörpers bzw. über die innere Mantelfläche der Ausnehmung hinaus ins Innere der Ausnehmung ragt. Dabei kann ein Ende des zusätzlichen Schraubelements mit einer geeigneten Kraft in einer Richtung quer oder schräg zur Mittelachse des Grundkörpers bzw. der Ausnehmung gegen das erste Ende des Koppelelements drücken, um dieses im Grundkörper bzw. in der Ausnehmung zu fixieren.

Gemäß einer Ausführungsform kann das Display über eine wiederlösbare Steckverbindung zur Stromversorgung und/oder zur Datenkommunikation mit mindestens einer der Komponenten im Hauptgehäuse elektrisch leitfähig verbunden sein. Die Steckverbindung kann beispielsweise durch bloßes Abnehmen des Displayhalters vom Hauptgehäuse und/oder vom Displaygehäuse gelöst werden. Umgekehrt ist es möglich, dass die Steckverbindung allein dadurch hergestellt wird, dass der Displayhalter bis in eine definierte Endposition ins Hauptgehäuse und/oder ins Displaygehäuse eingeführt wird. Die Steckverbindung kann beispielsweise als USB- oder USB-C-Steckverbindung ausgeführt sein und/oder vollständig im Inneren des Beatmungsgeräts, beispielsweise zumindest teilweise im Inneren des Hauptgehäuses und/oder zumindest teilweise im Inneren des Displayhalters und/oder zumindest teilweise im Inneren des Displaygehäuses, angeordnet sein. Dies ermöglicht es, das Display bei Bedarf schnell und einfach abzunehmen, beispielsweise um es woanders als direkt am Beatmungsgerät zu betreiben.

Gemäß einer Ausführungsform kann das Beatmungsgerät ferner eine Lichtquelle zum gezielten Beleuchten zumindest eines Abschnitts des Beatmungsgeräts und/oder einer Umgebung des Beatmungsgeräts umfassen. Unter "Lichtquelle" kann beispielsweise eine Glühbirne, eine Leuchtdiode oder eine Kombination aus mindestens zwei dieser Beispiele verstanden werden. Die Lichtquelle kann manuell oder automatisch, beispielsweise abhängig von einer gemessenen Helligkeit in der Umgebung des Beatmungsgeräts, in ihrer Helligkeit und/oder Farbe steuerbar sein. Im einfachsten Fall kann die Lichtquelle mittels eines geeigneten Schalters manuell ein- oder ausschaltbar sein. Die Lichtquelle kann beispielsweise in eine Außenwand des Hauptgehäuses, insbesondere in eine Gehäusefront des Hauptgehäuses, eingebaut sein.

Gemäß einer Ausführungsform kann das Beatmungsgerät neben dem Display (als einem Hauptdisplay) ein zusätzliches Display zum Anzeigen beatmungsrelevanter Informationen aufweisen. Das zusätzliche Display kann beispielsweise in eine Außenwand des Hauptgehäuses, insbesondere in eine Gehäusefront des Hauptgehäuses, eingebaut sein. Dementsprechend kann das zusätzliche Display eine deutlich kleinere Anzeigefläche als das Hauptdisplay haben. Das Beatmungsgerät kann ausgebildet sein, um Informationen, die für einen Beatmungsbetrieb erforderlich sind, zusätzlich auf dem zusätzlichen Display anzuzeigen. Dies ermöglicht es, den Beatmungsbetrieb auch dann aufrechtzuerhalten, wenn das Hauptdisplay aus irgendeinem Grund nicht zur Verfügung steht, etwa weil es in seiner Funktion aufgrund einer Störung beeinträchtigt ist oder im laufenden Betrieb getauscht wird.

### Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.
Fig. 1 zeigt ein Beatmungsgerät gemäß einer Ausführungsform der Erfindung.
Fig. 2 zeigt Details eines Displaygehäuses eines Beatmungsgeräts gemäß einer Ausführungsform der Erfindung.
Fig. 3 zeigt einen Querschnitt durch das Displaygehäuse aus Fig. 2 mit eingesetzter Displayeinheit entlang einer Schnittlinie A-A.
Fig. 4 zeigt einen Querschnitt durch einen ein Bedienelement aufweisenden Abschnitt einer Displayeinheit eines Beatmungsgeräts gemäß einer Ausführungsform der Erfindung.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

### Ausführungsformen der Erfindung

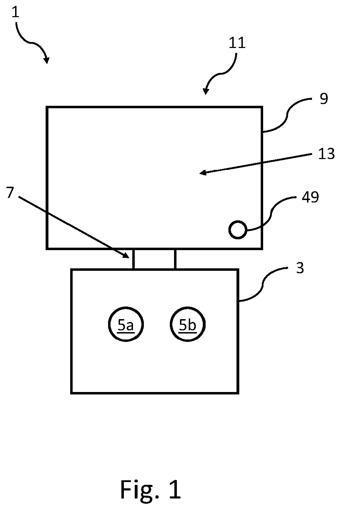

Fig. 1 zeigt ein Beatmungsgerät 1 zum invasiven und/oder nicht invasiven Beatmen eines Patienten.

Das Beatmungsgerät 1 umfasst ein Hauptgehäuse 3, das für einen Beatmungsbetrieb des Beatmungsgeräts 1 erforderliche Komponenten umgibt, z. B. eine oder mehrere Komponenten einer Steuerung, einer Sensorik und/oder einer Aktorik des Beatmungsgeräts 1. In diesem Beispiel weist das Hauptgehäuse 3 an seiner Gehäusefront einen Atemgasanschluss auf, der einen Atemgaseingang 5a zum Anschließen einer Atemgasquelle und einen Atemgasausgang 5b zum Anschließen eines zum Patienten führenden Schlauchsystems umfassen kann.

Das Hauptgehäuse 3 ist über einen Displayhalter 7 mechanisch mit einem Displaygehäuse 9 gekoppelt, insbesondere derart, dass ein Anwender das Displaygehäuse 9 in dessen Position und/oder Orientierung relativ zum Hauptgehäuse 3 anpassen und in der jeweiligen Position und/oder Orientierung fixieren kann.

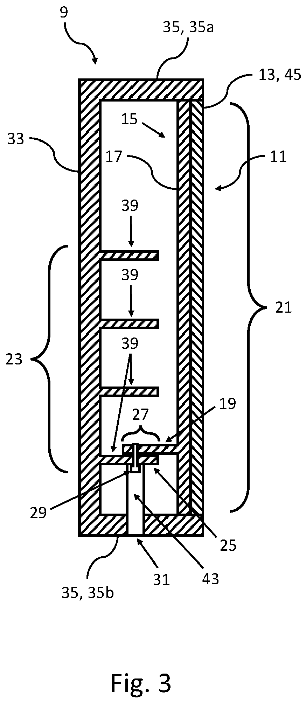

Darüber hinaus umfasst das Beatmungsgerät 1 eine Displayeinheit 11 mit einem Display 13 zum Anzeigen beatmungsrelevanter Informationen und einer Displayrückwand 15 (siehe Fig. 3), die eine an einer Rückseite des Displays 13 befestigte Grundplatte 17 und mehrere von der Grundplatte 17 abstehende erste Verschraubungsabschnitte 19 umfasst.

Das Displaygehäuse 9 ist schalenartig mit einer Schalenöffnung 21 ausgebildet. Die Displayeinheit 11 ist so vom Displaygehäuse 9 aufgenommen, dass das Display 13 die Schalenöffnung 21 mit seiner der Rückseite gegenüberliegenden Vorderseite teilweise, größtenteils oder vollständig abdeckt.

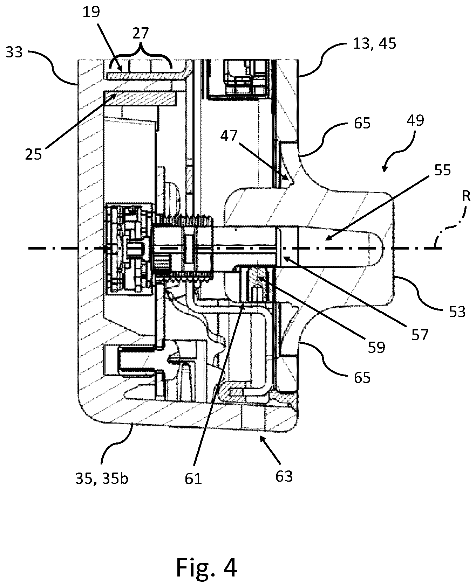

Das Displaygehäuse 9 weist in seinem Inneren eine mit einer Innenwand des Displaygehäuses 9 verbundene Versteifungsstruktur 23 zum Versteifen des Displaygehäuses 9 auf. Die Versteifungsstruktur 23 umfasst mehrere zweite Verschraubungsabschnitte 25, die so angeordnet sind, dass sie jeweils mindestens einem der ersten Verschraubungsabschnitte 19 in einem Überlappungsbereich 27 gegenüberliegen, wenn die Displayeinheit 11 - wie in Fig. 3 und Fig. 4 gezeigt - vollständig ins Displaygehäuse 9 eingeführt ist.

Die ersten Verschraubungsabschnitte 19 und die zweiten Verschraubungsabschnitte 25 sind in jedem der Überlappungsbereiche 27 mittels mindestens eines Schraubelements 29, hier in Form einer Schraube, miteinander verschraubt, sodass die Displayeinheit 11 am Displaygehäuse 9 fixiert und somit zusammen mit dem Displaygehäuse 9 verstellbar ist. Dabei befindet sich jedes der Schraubelemente 29 vollständig im Inneren des Displaygehäuses 9.

Des Weiteren sind in einer Außenwand des Displaygehäuses 9 mehrere Zugangsöffnungen 31 so ausgebildet, dass jedes der Schraubelemente 29 über eine der Zugangsöffnungen 31 von außerhalb des Displaygehäuses 9 für ein Schraubwerkzeug, beispielsweise einen Schraubenzieher, zugänglich ist. Ein Abstand D einer jeden der Zugangsöffnungen 31 zu einem der Zugangsöffnung 31 zugewandten Ende des jeweiligen Schraubelements 29 kann beispielsweise mindestens 1 cm, mindestens 2 cm, mindestens 5 cm oder mindestens 10 cm betragen.

In diesem Beispiel ist jedes der Schraubelemente 29 im jeweiligen Überlappungsbereich 27 durch ein Loch in einem der ersten Verschraubungsabschnitte 19 und durch ein weiteres Loch in einem der zweiten Verschraubungsabschnitte 25 geführt und mit einer Schraubenmutter (nicht gezeigt), beispielsweise in Form einer Einpressmutter, am jeweiligen zweiten Verschraubungsabschnitt 25 verschraubt.

Das Displaygehäuse 9 kann durch einen Schalenboden 33 und eine mit dem Schalenboden 33 verbundene und diesen umlaufende Schalenseitenwand 35 begrenzt sein. Dabei kann die Schalenöffnung 21 durch einen vom Schalenboden 33 abgewandten umlaufenden Rand der Schalenseitenwand 35 begrenzt sein.

Dementsprechend kann das Displaygehäuse 9 in Richtung seiner Höhe H einerseits durch einen oberen Abschnitt 35a der Schalenseitenwand 35 und andererseits durch einen unteren Abschnitt 35b der Schalenseitenwand 35 sowie in Richtung seiner Breite B einerseits durch einen linken Abschnitt 35c der Schalenseitenwand 35 und andererseits durch einen rechten Abschnitt 35d der Schalenseitenwand 35 begrenzt sein.

Dabei können sämtliche Zugangsöffnungen 31 in mindestens einem der vier Abschnitte der Schalenseitenwand 35 ausgebildet sein. In diesem Beispiel sind alle vier Zugangsöffnungen 31 im unteren Abschnitt 35b ausgebildet.

Die Versteifungsstruktur 23 kann eine Mehrzahl länglicher Versteifungsrippen umfassen, die jeweils einerseits mit der Schalenseitenwand 35 und andererseits mit dem Schalenboden 33 verbunden sein können.

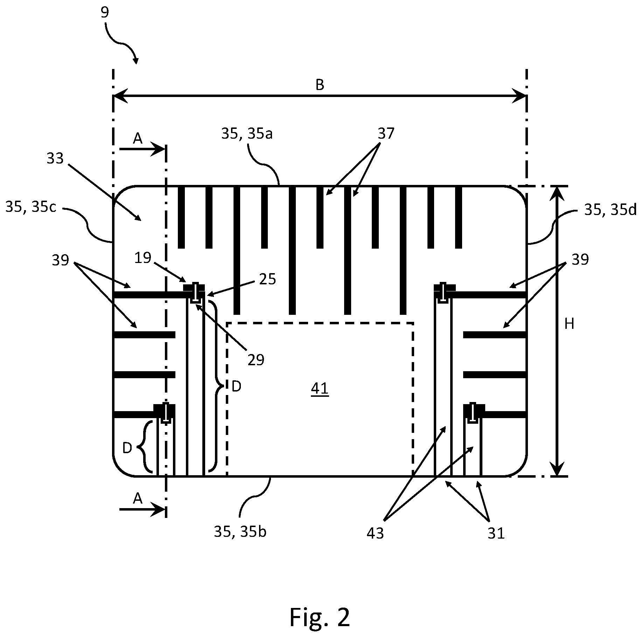

In diesem Beispiel umfassen die Versteifungsrippen eine Mehrzahl parallel zueinander angeordneter erster Versteifungsrippen 37 und eine Mehrzahl parallel zueinander angeordneter zweiter Versteifungsrippen 39, die sich in ihrer jeweiligen Längsrichtung von den ersten Versteifungsrippen 37 unterscheiden. Beispielsweise können die ersten Versteifungsrippen 37, wie in Fig. 2 gezeigt, in ihrer Längsrichtung betrachtet jeweils orthogonal zu den zweiten Versteifungsrippen 39 ausgerichtet sein. Alternativ ist eine schräge Ausrichtung möglich.

Die ersten Versteifungsrippen 37 sind jeweils einerseits mit dem oberen Abschnitt 35a und andererseits mit dem Schalenboden 33 verbunden. Einige der zweiten Versteifungsrippen 39 sind jeweils einerseits mit dem linken Abschnitt 35c und andererseits mit dem Schalenboden 33 verbunden, wobei die restlichen zweiten Versteifungsrippen 39 jeweils einerseits mit dem rechten Abschnitt 35d und andererseits mit dem Schalenboden 33 verbunden sind. Möglich sind aber auch andere Verbindungen. Beispielsweise können die Versteifungsrippen 37, 39 auch miteinander verbunden sein und/oder einander gegenüberliegende Abschnitte der Schalenseitenwand 35 miteinander verbinden.

Wie in Fig. 2 zu sehen, beträgt die jeweilige Länge der Versteifungsrippen 37, 39 deutlich weniger als 50 % der Höhe H bzw. der Breite B, sodass ein bestimmter Bereich des Displaygehäuses 9, genauer des Schalenbodens 33, frei von Versteifungsrippen ist. Dieser ausgesparte Bereich kann als ein Displayaufnahmebereich 41 fungieren, von dem ein bestimmter rückseitiger Abschnitt der Displayeinheit 11 aufgenommen ist, wenn diese vollständig ins Displaygehäuse 9 eingeführt ist. Dies ermöglicht eine besonders flache Bauform des Displaygehäuses 9.

In diesem Beispiel sind die insgesamt vier zweiten Verschraubungsabschnitte 25 verschiedene Abschnitte von vier der zweiten Versteifungsrippen 39, genauer von zwei linken und zwei rechten zweiten Versteifungsrippen 39.

Zusätzlich können im Inneren des Displaygehäuses 9 mehrere Schächte 43 zum Führen des Schraubwerkzeugs ausgebildet sein. In diesem Beispiel erstrecken sich die Schächte 43 jeweils geradlinig in Richtung der Höhe H ausgehend von einer der Zugangsöffnungen 31 hin zu einem der Schraubelemente 29, genauer hin zu einem der jeweiligen Zugangsöffnung 31 zugewandten Ende von einem der Schraubelemente 29, hier einem Schraubenkopf.

Zweckmäßigerweise ist eine Schachtwand eines jeden der Schächte 43 so ausgebildet, dass ein im Schacht 43 befindliches Schraubelement 29 bei der Montage oder Demontage der Displayeinheit 11 nicht versehentlich aus dem Schacht 43 heraus ins Innere des Displaygehäuses 9 fallen kann.

Die Schachtwand kann einen Abschnitt des Schalenbodens 33 und/oder einander gegenüberliegende Abschnitte von zwei der zueinander parallel ausgerichteten Versteifungsrippen 37 bzw. 39 umfassen. Zusätzlich oder alternativ kann die Schachtwand einen Abschnitt der Displayrückwand 15, beispielsweise der Grundplatte 17, und/oder einen Abschnitt der Schalenseitenwand 35 umfassen.

Je nach Ausführungsform kann das Display 13 an seiner Vorderseite eine Displayscheibe 45 aus Glas und/oder Kunststoff aufweisen. Die Displayscheibe 45 kann die Schalenöffnung 21 größtenteils oder vollständig abdecken.

Wie in Fig. 4 zu sehen, kann die Displayscheibe 45 ein durch einen (vollständig) umlaufenden Rand begrenztes Scheibenloch 47 aufweisen, das eine Vorderseite der Displayscheibe 45 mit deren Rückseite verbindet. Im und/oder am Scheibenloch 47 kann ein Bedienelement 49 zum Bedienen des Displays 13 und/oder des Beatmungsgeräts 1 relativ zur Displayscheibe 45 dreh- und/oder verschiebbar gelagert sein.

In diesem Beispiel ist das Bedienelement 49 als ein relativ zur Displayscheibe 45 um eine Drehachse R drehbarer Drehknopf ausgeführt. Dabei kann das Bedienelement 49 einen zylinderförmigen Grundkörper 53 mit einer becher- oder buchsenförmigen Ausnehmung 55 umfassen. Die Drehachse R stimmt hier beispielhaft mit der Mittelachse des Grundkörpers 53 überein. Der Grundkörper 53 kann durch das beispielsweise kreisrunde Scheibenloch 47 geführt sein, sodass er in Richtung der Drehachse R betrachtet beidseitig von der Displayscheibe 45, d. h. sowohl von deren Vorderseite als auch von deren Rückseite, absteht. In die Ausnehmung 55 kann ein erstes Ende eines stabförmigen Koppelelements 57 eingeführt und mittels eines zusätzlichen Schraubelements 59 darin fixiert sein. Ein zweites Ende des Koppelelements 57 kann hingegen im Inneren des Displaygehäuses 9, beispielsweise am Schalenboden 33, um die Drehachse R drehbar gelagert sein.

Das zusätzliche Schraubelement 59, beispielsweise eine Madenschraube, ist hier in einer Richtung quer zur Drehachse R in eine Durchgangsöffnung 61, die eine äußere Mantelfläche des Grundkörpers 53 mit einer inneren Mantelfläche der Ausnehmung 55 verbindet, geschraubt.

Zudem kann in der Außenwand des Displaygehäuses 9 eine zusätzliche Zugangsöffnung 63 so ausgebildet sein, dass das zusätzliche Schraubelement 59 in einer bestimmten Drehposition des Bedienelements 49 (oder des Grundkörpers 53) über die zusätzliche Zugangsöffnung 63 von außerhalb des Displaygehäuses 9 für ein Schraubwerkzeug zugänglich ist.

In dem in Fig. 4 gezeigten Beispiel ist die zusätzliche Zugangsöffnung 63 wie die übrigen Zugangsöffnungen 31 im unteren Abschnitt 35b der Schalenseitenwand 35 ausgebildet. Dementsprechend ist das zusätzliche Schraubelement 59 für das Schraubwerkzeug zugänglich, wenn das Bedienelement 49 in eine Position gedreht ist, in der das zusätzliche Schraubelement 59 mit einem seiner Enden der zusätzlichen Zugangsöffnung 63 vertikal gegenüberliegt, d. h., wenn die zusätzliche Zugangsöffnung 63 und das zusätzliche Schraubelement 59 übereinanderliegen, insbesondere derart, dass die Mittelachse der zusätzlichen Zugangsöffnung 63 und die Längsachse des zusätzlichen Schraubelements 59 auf einer gemeinsamen Geraden oder auf zueinander parallelen Geraden mit entsprechend geringem Abstand voneinander liegen. In dieser Position kann ein Abstand der zusätzlichen Zugangsöffnung 63 zu einem der zusätzlichen Zugangsöffnung 63 zugewandten Ende des zusätzlichen Schraubelements 59 beispielsweise mindestens 1 cm, mindestens 2 cm, mindestens 5 cm oder mindestens 10 cm betragen.

Das Bedienelement 49 kann zusätzlich einen Kragen 65 umfassen, der von einer äußeren Mantelfläche des Grundkörpers 53 absteht und diese in deren Umfangsrichtung ringförmig umgibt. Der Kragen 65 kann eine an eine ringförmige Innenkontur des Scheibenlochs 47 angepasste ringförmige Außenkontur aufweisen und derart ins Scheibenloch 47 eingeführt sein, dass seine Außenkontur zumindest teilweise von der Innenkontur des Scheibenlochs 47 umgeben ist. Die Innenkontur und die Außenkontur können in ihrem Durchmesser derart aufeinander abgestimmt sein, dass sie durch einen (beispielsweise ebenfalls ringförmigen) Luftspalt voneinander getrennt sind. Der Luftspalt kann zusätzlich mit einem geeigneten Dichtmaterial fluiddicht verschlossen sein. Alternativ ist es möglich, dass sich die Innenkontur und die Außenkontur an einer ringförmigen Berührungsfläche berühren, insbesondere derart, dass über die Berührungsfläche kein Wasser ins Innere des Displaygehäuses 9 eindringen kann. Zudem kann der Kragen 65 als axialer Anschlag zum Positionieren des Grundkörpers 53 in Richtung der Drehachse R relativ zur Displayscheibe 45 ausgebildet sein.

Um zu verhindern, dass sich Schmutz oder Flüssigkeit am Übergang zwischen dem Kragen 65 und der Displayscheibe 45 sammelt, kann der Kragen 65 so geformt sein, dass er - in Richtung der Drehachse R betrachtet - bündig mit der einem Betrachter des Displays 13 zugewandten Vorderseite der Displayscheibe 45 abschließt, d. h., der Übergang kann im Wesentlichen stufenlos ausgebildet sein.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

### Liste der Bezugszeichen

- 1: Beatmungsgerät
- 3: Hauptgehäuse
- 5a: Atemgaseingang
- 5b: Atemgasausgang
- 7: Displayhalter
- 9: Displaygehäuse
- 11: Displayeinheit
- 13: Display
- 15: Displayrückwand
- 17: Grundplatte
- 19: erster Verschraubungsabschnitt
- 21: Schalenöffnung
- 23: Versteifungsstruktur
- 25: zweiter Verschraubungsabschnitt
- 27: Überlappungsbereich
- 29: Schraubelement
- 31: Zugangsöffnung
- 33: Schalenboden
- 35: Schalenseitenwand
- 35a: oberer Abschnitt
- 35b: unterer Abschnitt
- 35c: linker Abschnitt
- 35d: rechter Abschnitt
- 37: erste Versteifungsrippe
- 39: zweite Versteifungsrippe
- 41: Displayaufnahmebereich
- 43: Schacht
- 45: Displayscheibe
- 47: Scheibenloch
- 49: Bedienelement
- 53: Grundkörper
- 55: Ausnehmung
- 57: Koppelelement
- 59: zusätzliches Schraubelement
- 61: Durchgangsöffnung
- 63: zusätzliche Zugangsöffnung
- 65: Kragen
- B: Breite
- D: Abstand
- H: Höhe
- R: Drehachse

## Patentansprüche

1. Beatmungsgerät (1), umfassend:
ein Hauptgehäuse (3) zum Aufnehmen von Komponenten, die für einen Beatmungsbetrieb des Beatmungsgeräts (1) erforderlich sind, wobei das Hauptgehäuse (3) einen Atemgasanschluss (5a, 5b) zum Anschließen einer Atemgasquelle und/oder eines zu einem Patienten führenden Schlauchsystems umfasst;
eine Displayeinheit (11) mit einem Display (13) zum Anzeigen beatmungsrelevanter Informationen und einer Displayrückwand (15), die eine an einer Rückseite des Displays (13) befestigte Grundplatte (17) und mehrere von der Grundplatte (17) abstehende erste Verschraubungsabschnitte (19) umfasst;
ein Displaygehäuse (9), das schalenartig mit einer Schalenöffnung (21) zum Einführen der Displayeinheit (11) ausgebildet ist, wobei die Displayeinheit (11) so vom Displaygehäuse (9) aufgenommen ist, dass das Display (13) die Schalenöffnung (21) mit seiner der Rückseite gegenüberliegenden Vorderseite zumindest teilweise abdeckt;
einen Displayhalter (7), der das Displaygehäuse (9) mechanisch mit dem Hauptgehäuse (3) koppelt;
wobei das Displaygehäuse (9) in seinem Inneren eine mit einer Innenwand des Displaygehäuses (9) verbundene Versteifungsstruktur (23) zum Versteifen des Displaygehäuses (9) aufweist, wobei die Versteifungsstruktur (23) mehrere zweite Verschraubungsabschnitte (25) umfasst, wobei die zweiten Verschraubungsabschnitte (25) so angeordnet sind, dass sie jeweils mindestens einem der ersten Verschraubungsabschnitte (19) in einem Überlappungsbereich (27) gegenüberliegen, wenn die Displayeinheit (11) vollständig ins Displaygehäuse (9) eingeführt ist, wobei die ersten Verschraubungsabschnitte (19) und die zweiten Verschraubungsabschnitte (25) in jedem der Überlappungsbereiche (27) mittels mindestens eines Schraubelements (29) miteinander verschraubt sind, sodass die Displayeinheit (11) am Displaygehäuse (9) fixiert ist, wobei jedes der Schraubelemente (29) vollständig im Inneren des Displaygehäuses (9) angeordnet ist, wobei in einer Außenwand des Displaygehäuses (9) mehrere Zugangsöffnungen (31) so ausgebildet sind, dass jedes der Schraubelemente (29) über eine der Zugangsöffnungen (31) von außerhalb des Displaygehäuses (9) für ein Schraubwerkzeug zugänglich ist.

2. Beatmungsgerät (1) nach Anspruch 1,
wobei ein Abstand (D) einer jeden der Zugangsöffnungen (31) zu einem der Zugangsöffnung (31) zugewandten Ende des jeweiligen Schraubelements (29) mindestens 1 cm, mindestens 2 cm, mindestens 5 cm oder mindestens 10 cm beträgt.

3. Beatmungsgerät (1) nach einem der vorhergehenden Ansprüche,
wobei die Schraubelemente (29) mehrere Schrauben (29) umfassen, wobei jede der Schrauben (29) durch ein Loch in einem der ersten Verschraubungsabschnitte (19) und/oder durch ein Loch in einem der zweiten Verschraubungsabschnitte (25) geführt ist und/oder mit einer Schraubenmutter, die an einem der ersten Verschraubungsabschnitte (19) und/oder an einem der zweiten Verschraubungsabschnitte (25) angeordnet ist, verschraubt ist.

4. Beatmungsgerät (1) nach einem der vorhergehenden Ansprüche,
wobei das Displaygehäuse (9) einen Schalenboden (33) und eine mit dem Schalenboden (33) verbundene Schalenseitenwand (35) umfasst, wobei die Schalenöffnung (21) durch einen umlaufenden Rand der Schalenseitenwand (35) begrenzt ist, wobei das Displaygehäuse (9) in Richtung seiner Höhe (H) einerseits durch einen oberen Abschnitt (35a) der Schalenseitenwand (35) und andererseits durch einen unteren Abschnitt (35b) der Schalenseitenwand (35) und/oder in Richtung seiner Breite (B) einerseits durch einen linken Abschnitt (35c) der Schalenseitenwand (35) und andererseits durch einen rechten Abschnitt (35d) der Schalenseitenwand (35) begrenzt ist, wobei die Zugangsöffnungen (31) in mindestens einem der Abschnitte (35a, 35b, 35c, 35d) der Schalenseitenwand (35) ausgebildet sind.

5. Beatmungsgerät (1) nach Anspruch 4,
wobei die Zugangsöffnungen (31) außerhalb des oberen Abschnitts (35a) ausgebildet sind; und/oder
wobei die Zugangsöffnungen (31) nur in einem der Abschnitte (35a, 35b, 35c, 35d) der Schalenseitenwand (35), insbesondere nur im unteren Abschnitt (35b), ausgebildet sind.

6. Beatmungsgerät (1) nach Anspruch 4 oder 5,
wobei die Versteifungsstruktur (23) eine Mehrzahl länglicher Versteifungsrippen (37, 39) umfasst, wobei die Versteifungsrippen (37, 39) jeweils einerseits mit der Schalenseitenwand (35) und andererseits mit dem Schalenboden (33) verbunden sind, wobei die Versteifungsrippen (37, 39) eine Mehrzahl parallel zueinander angeordneter erster Versteifungsrippen (37) und eine Mehrzahl parallel zueinander angeordneter zweiter Versteifungsrippen (39) umfassen, wobei die ersten Versteifungsrippen (37) in ihrer Längsrichtung betrachtet jeweils orthogonal und/oder schräg zu den zweiten Versteifungsrippen (39) ausgerichtet sind.

7. Beatmungsgerät (1) nach Anspruch 6,
wobei die ersten Versteifungsrippen (37) jeweils einerseits mit dem oberen Abschnitt (35a) und/oder dem unteren Abschnitt (35b) der Schalenseitenwand (35) und andererseits mit dem Schalenboden (33) verbunden sind; und/oder
wobei die zweiten Versteifungsrippen (39) jeweils einerseits mit dem linken Abschnitt (35c) und/oder dem rechten Abschnitt (35d) der Schalenseitenwand (35) und andererseits mit dem Schalenboden (33) verbunden sind; und/oder
wobei mindestens eine der ersten Versteifungsrippen (37) zusätzlich mit mindestens einer der zweiten Versteifungsrippen (39) verbunden ist.

8. Beatmungsgerät (1) nach Anspruch 6 oder 7,
wobei eine jeweilige Länge der Versteifungsrippen (37, 39) höchstens 30 %, höchstens 50 % oder höchstens 70 % der Höhe (H) und/oder der Breite (B) des Displaygehäuses (9) beträgt; und/oder
wobei das Displaygehäuse (9) in seinem Inneren einen von den Versteifungsrippen (37, 39) freien Displayaufnahmebereich (41) zum Aufnehmen zumindest eines Abschnitts der Displayeinheit (11) aufweist.

9. Beatmungsgerät (1) nach einem der Ansprüche 6 bis 8,
wobei mindestens einer oder jeder der zweiten Verschraubungsabschnitte (25) ein Abschnitt von einer der Versteifungsrippen (37, 39) ist.

10. Beatmungsgerät (1) nach einem der vorhergehenden Ansprüche,
wobei im Inneren des Displaygehäuses (9) mehrere Schächte (43) zum Führen des Schraubwerkzeugs ausgebildet sind, wobei sich jeder der Schächte (43) ausgehend von einer der Zugangsöffnungen (31) hin zu dem der Zugangsöffnung (31) zugewandten Ende des jeweiligen Schraubelements (29) erstreckt, wobei eine Schachtwand eines jeden der Schächte (43) so ausgebildet ist, dass ein im Schacht (43) befindliches Schraubelement (29) nicht aus dem Schacht (43) heraus ins Innere des Displaygehäuses (9) fallen kann.

11. Beatmungsgerät (1) nach Anspruch 10 rückbezogen auf Anspruch 6,
wobei die Schachtwand einen Abschnitt von mindestens einer der Versteifungsrippen (37, 39) umfasst.

12. Beatmungsgerät (1) nach einem der vorhergehenden Ansprüche,
wobei das Display (13) an seiner Vorderseite eine Displayscheibe (45) aus Glas und/oder Kunststoff aufweist, wobei die Displayscheibe (45) die Schalenöffnung (21) größtenteils oder vollständig abdeckt;
wobei die Displayeinheit (11) ferner ein Bedienelement (49) umfasst, das an und/oder in einem Scheibenloch (47) in der Displayscheibe (45) relativ zur Displayscheibe (45) dreh- und/oder verschiebbar gelagert ist.

13. Beatmungsgerät (1) nach Anspruch 12,
wobei das Bedienelement (49) mittels eines zusätzlichen Schraubelements (59) an einem ersten Ende eines länglichen Koppelelements (57) fixiert ist und ein zweites Ende des Koppelelements (57) dreh- und/oder verschiebbar am und/oder im Displaygehäuse (9) gelagert ist, wobei das zusätzliche Schraubelement (59) vollständig im Inneren des Displaygehäuses (9) angeordnet ist, wobei in der Außenwand des Displaygehäuses (9) eine zusätzliche Zugangsöffnung (63) so ausgebildet ist, dass das zusätzliche Schraubelement (59) in einer bestimmten Position und/oder Orientierung des Bedienelements (49) relativ zur Displayscheibe (45) über die zusätzliche Zugangsöffnung (63) von außerhalb des Displaygehäuses (9) für ein Schraubwerkzeug zugänglich ist.

14. Beatmungsgerät (1) nach Anspruch 13,
wobei das erste Ende des Koppelelements (57) in eine Ausnehmung (55) des Bedienelements (49) eingeführt ist und mittels des zusätzlichen Schraubelements (59) in der Ausnehmung (55) fixiert ist; und/oder
wobei das Bedienelement (49) einen zylinderförmigen Grundkörper (53) und einen von einer äußeren Mantelfläche des Grundkörpers (53) abstehenden Kragen (65) mit einer an eine Innenkontur des Scheibenlochs (47) angepassten Außenkontur umfasst, wobei der Grundkörper (53) durch das Scheibenloch (47) geführt ist, sodass er beidseitig von der Displayscheibe (45) absteht, wobei der Kragen (65) ins Scheibenloch (47) eingeführt ist, sodass die Außenkontur zumindest teilweise von der Innenkontur umgeben ist.

15. Beatmungsgerät (1) nach Anspruch 14,
wobei der Kragen (65) bündig mit der Vorderseite des Displays (13) abschließt; und/oder
wobei ein Spalt zwischen dem Kragen (65) und der Displayscheibe (45), insbesondere zwischen der Innenkontur und der Außenkontur, fluiddicht verschlossen ist; und/oder
wobei sich der Kragen (65) und die Displayscheibe (45) an einer ringförmigen Berührungsfläche berühren; und/oder
wobei sich die Innenkontur und die Außenkontur an einer ringförmigen Berührungsfläche berühren; und/oder
wobei das zusätzliche Schraubelement (59) in eine Durchgangsöffnung (61), die die äußere Mantelfläche des Grundkörpers (53) mit einer inneren Mantelfläche des Grundkörpers (53) und/oder mit einer inneren Mantelfläche der Ausnehmung (55) verbindet, eingeführt ist.
